# EUROPEAN PATENT APPLICATION

(11) **EP 3 488 780 A1**
(43) Date of publication of application: **29.05.2019**
(21) Application number: 18206124.2
(22) Date of filing: 14.11.2018
(51) Int. Cl.: A61B 5/11, A61B 5/00

(54) **HEALTHCARE MONITORING SYSTEM**

(30) Priority: 15.11.2017 TW 10639553
(71) Applicant: H.P.B Optoelectronic Co., Ltd., Taichung City 428 (TW)
(72) Inventor: Hsu, Hsuan-Yueh, 428 Taichung City (TW); Ruan, Yu-Sheng, 428 Taichung City (TW); Chang, Sheng-Chung, 428 Taichung City (TW)
(74) Representative: Gee, Steven William

(57) **Abstract**

A healthcare monitoring system for health caring and space monitoring that determines movements or existence of a care recipient by utilizing visual images to detect variation in shape of an optical pattern is provided. The system includes a light source unit, an image capturing unit, an image processing unit and a warning unit. By capturing the shape variation of the optical pattern emitted by the light source unit provided around a lying equipment via the image capturing unit, the system effectively determines motions of the care recipient lying on the lying equipment, such as motions of getting up, turning over and hand waving, in addition to lying flat, and detects activities of care providers or irrelevant persons around the lying equipment, thereby having an advantage that the activities of the care recipient lying on the lying equipment and the condition around the lying equipment can be monitored comprehensively.

## Description

### CROSS-REFERENCE TO RELATED APPLICATION

This application claims the priority of Taiwanese patent application No. 106139553, filed on November 15, 2017, which is incorporated herewith by reference.

### BACKGROUND OF THE INVENTION

### 1. Field of the Invention

The present invention relates generally to a healthcare monitoring system, and more particularly to a system for health caring and space monitoring which determines movements or existence of a care recipient by utilizing visual images to detect variation in shape of an optical pattern.

### 2. The Prior Arts

With an increasing of aging population and advancement in medical technology and equipment, the fact that many medical care activities which required treatments in hospital facilities in the past are now possible to be completed in residential environments of care recipients, such as elderly or patients etc., has led to prosper developments in fields and industries relevant to domiciliary care. It is common for domiciliary care and nursing homes to employ care workers to take care of care recipients. Yet, there is still a large population of elderly who are not ill but stay at home alone most of the time and independent elderly who live alone by themselves. These non-ill or independent elderly people have no need to involve a care provider for care support, but they do inevitably encounter mental deterioration, physical weakness, and decline in mobility due to their old age, which result in occasionally happened domiciliary events such as falls or more serious events such as fainting or myocardial infarction.

Relevant research has been conducted to solve the above problems. For example, some elderly care systems are equipped with a tilt sensor to be worn on the elderly, such that a tilt signal can be detected when the elderly fall and relevant warning messages would be sent to mobile communication devices of their family members to call attention to immediate aid. Yet, in this case, if the elderly forget to wear the tilt sensor or if the tilt sensor falls off from the elderly, it is impossible to effectively monitor the elderly's falling events. Taiwanese Patent M549405 titled "a detector and an eldercare system containing the same" was developed to solve this problem. The patent comprises a sensor module, a process module and a wireless transmission module. The sensor module detects whether there is an object passing in the front thereof; the process module is connected with the sensor module; and the wireless transmission module is connected with the process module, wherein when the sensor module detects no object passing in the front of the sensor module within a predetermined time interval, the process module generates a warning signal which can be sent via the wireless transmission module. The patent primarily detects whether there is an object passing in the front of the sensor module by means of infrared sensing, and drives the process module to activate an illumination module to provide a function similar to a night light to effectively prevent elderly from accidental events such as falling due to insufficient light at night. If the infrared sensor module cannot detect the object passing in the front thereof within the predetermined time interval, the process module is driven to generate a warning signal to be sent to mobile communication devices of the elderly's family members via a cloud server in order to notify family members for immediate aid. Nevertheless, the sensor presented by infrared sensor is only capable of detecting certain set motions at a set position. Since there are no images provided for observation, the fact that the elderly does not pass through the detected area of the infrared sensor does not necessary represent that an accidental event has occurred; sending a warning signal at this time would be a misleading action. Therefore, it is thus indeed crucial for developers and researchers of the related industries of the healthcare monitoring system to overcome the problems in the prior arts and develop a new software design of the healthcare monitoring system which effectively determines a care recipient's motions, such as getting up, turning over, and hand waving, in addition to lying flat, on the bed, and detects relevant activities of care providers or irrelevant persons around the patient's bed.

### SUMMARY OF THE INVENTION

In view of the fact that there are still many deficiencies in conventional healthcare systems for actual implementation, the inventor of the present invention proposes the present invention to improve conventional healthcare systems based on his professional knowledge and years of practical experience.

The primary objective of the present invention is to provide a healthcare monitoring system, in particular a system for health caring and space monitoring which determines movements or existence of a care recipient by utilizing visual images to detect variation in shape of an optical pattern. By capturing the shape variation of the optical pattern emitted by the light source unit provided at the surrounding of a lying equipment via the image capturing unit, the present invention effectively determines motions of the care recipient lying on the lying equipment, such as motions of getting up, turning over and hand waving, in addition to lying flat, and detects activities of care providers or irrelevant persons around the lying equipment. Thereby the present invention overcomes the disadvantages that conventional care monitoring executed by an infrared transceiver or a gravity sensor is only capable of detecting certain set motions and that by a wearable sensor is merely allowed to analyze vital signs of the care recipients, and provides an advantage that the activities of the care recipient on the lying equipment as well as the condition around the lying equipment can be monitored comprehensively.

In order to achieve the above-mentioned objective, the present invention proposed a healthcare monitoring system at least comprising a light source unit, an image capturing unit, an image processing unit and a warning unit. The light source unit is provided at a side of a lying equipment, the light source unit emits a light beam including at least an optical pattern toward the lying equipment, wherein the optical pattern is varied as projecting onto at least an object. The image capturing unit is provided at a side of the light source unit, the image capturing unit includes an optical lens and an optical sensor connected to the optical lens, wherein the optical lens continuously captures a plurality of images of the object and the optical pattern to form the images onto the optical sensor. The image processing unit is electrically connected to the image capturing unit, the image processing unit includes a reception module and a determination module electrically connected to the reception module. The reception module receives the plurality of images captured by the image capturing unit and defines the image captured at an initial time to be an initial image. The determination module compares the image captured at a subsequent time with the initial image, when the result of comparing indicates the images being inconsistent, the determination module sends a signal. The warning unit receives the signal sent by the determination unit and sends a warning signal.

The healthcare monitoring system as described above, further comprising a storage unit electrically connected to the image capturing unit to store the plurality of images captured by the image capturing unit.

The healthcare monitoring system as described above, the lying equipment is one equipment selected from the group consisting of a home bed, a medical bed, an electric bed, a care bed, a flat bed, a Japanese bed, an air-cushioned bed, a futon, a baby cot, a sofa and a long bench.

The healthcare monitoring system as described above, wherein the light source unit is one device selected from the group consisting of a light-emitting diode, a laser diode, and a light-emitting element for outputting a light beam.

The healthcare monitoring system as described above, wherein the light beam has a wavelength above 800nm. Preferably, the light beam has a wavelength between 1000nm to 1500nm, which satisfies an optical window in biological tissue.

The healthcare monitoring system as described above, wherein the optical pattern is one form selected from the group consisting of a grid, a line, a cross, regular arranged points, and irregular arranged points.

The healthcare monitoring system as described above, wherein the object is a dynamic object or a stationary object, or a combination of both.

The healthcare monitoring system as described above, wherein the optical lens is one device selected from the group consisting of a fisheye lens, a wide-angle lens and a standard lens.

The healthcare monitoring system as described above, wherein the optical sensor is a charge coupled device (CCD) or a complementary metal-oxide-semiconductor (CMOS).

The healthcare monitoring system as described above, wherein the warning unit is one device selected from the group consisting of an alarm, a display and a mobile communication device.

The healthcare monitoring system as described above, wherein the warning signal is presented by the warning unit as one selected from the group consisting of a sound, a light, an image, a vibration and a combination thereof.

The healthcare monitoring system as described above, further comprising a cloud server, wherein the determination module, the cloud server and the warning unit are interconnected with each other by a wireless transmission.

The healthcare monitoring system as described above, wherein the wireless transmission is one selected from the group consisting of a bluetooth communication, an infrared communication, a near field communication (NFC), a wireless local area network (WLAN), a wireless gigabit alliance (WiGig), a ZigBee, a wireless universal serial bus (wireless USB), an ultra-wideband (UWB), a wireless fidelity (Wi-Fi), and a combination thereof.

The healthcare monitoring system as described above, wherein the light source unit is a flashing light source of which a flashing frequency is the same as an image-capturing frequency of the image capturing unit.

The healthcare monitoring system as described above, wherein the image processing unit may further compare a former image and a latter image captured by the image capturing unit and acquire the difference between the two images to determine variation of the optical pattern.

Accordingly, by capturing the shape variation of the optical pattern emitted by the light source unit provided at the surrounding of the lying equipment via the image capturing unit, the present invention effectively determines motions of the care recipient lying on the lying equipment, such as motions of getting up, turning over and hand waving, in addition to lying flat, and detects activities of care providers or irrelevant persons around the lying equipment. Thereby, the present invention has advantages that the activities of the care recipient lying on the lying equipment as well as the condition around the lying equipment can be monitored comprehensively, and that the set up cost of the system can be reduced. Besides, by utilizing the variation in shape of the optical pattern, the present invention overcomes the disadvantages that conventional care monitoring executed by an infrared transceiver or a gravity sensor is only capable of detecting certain set motions and that by a wearable sensor is merely allowed to analyze the vital signs of the care recipients, thereby having advantages that the motions and conditions of all dynamic objects within a spacious room can be monitored and that the monitoring is free from errors. Finally, by setting up hardware in which the light source is capable of emitting the optical pattern in infrared light, the healthcare monitoring system of the present invention provides effective light source for the image capturing unit at a midnight environment where no light is given for illumination, such that the variation in shape of the optical pattern can be captured by means of visual images, thereby having advantages in achieving all day monitoring of the care recipient's actions and conditions, and executing all day security monitoring in the room.

### BRIEF DESCRIPTION OF THE DRAWINGS

Fig. 1 is a schematic block diagram of the healthcare monitoring system according to a preferred embodiment of the present invention; and
Fig. 2 is a configuration diagram of the healthcare monitoring system according to a preferred embodiment of the present invention.

### DETAILED DESCRIPTION OF THE PREFERRED EMBODIMENT

The accompanying drawings are included to provide a further understanding of the invention, and are incorporated in and constitute a part of this specification. The drawings illustrate embodiments of the invention and, together with the description, serve to explain the principles of the invention.

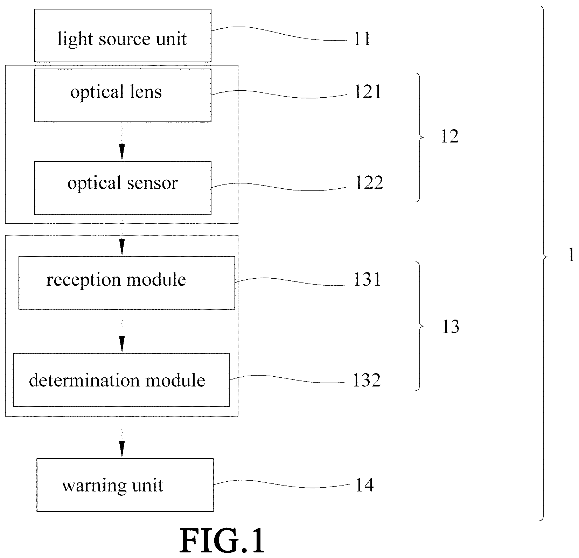

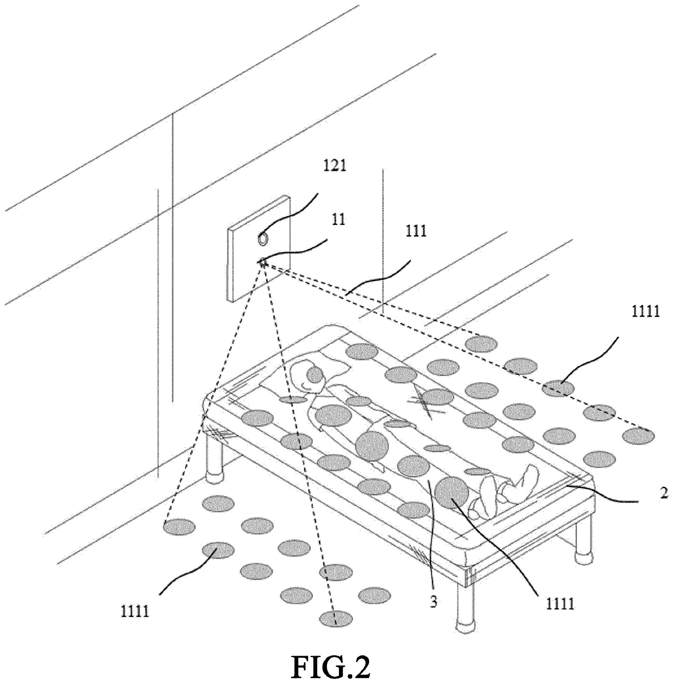

First, refer to Fig. 1 and Fig. 2, which illustrate a schematic block diagram and a configuration diagram of the healthcare monitoring system according to a preferred embodiment of the present invention, respectively. The healthcare monitoring system according to the present invention at least comprises a light source unit 11, an image capturing unit 12, an image processing unit 13 and a warning unit 14.

The light source unit 11 is provided at a side of a lying equipment 2, the light source unit 11 emits a light beam 111 including at least an optical pattern 1111 toward the lying equipment 2, wherein the optical pattern 1111 is varied as projecting onto at least an object 3. In addition, the lying equipment 2 is one selected from the group consisting of a home bed, a medical bed, an electric bed, a care bed, a flat bed, a Japanese bed, an air-cushioned bed, a futon, a baby cot, a sofa and a long bench. The light source unit 11 is one device selected from the group consisting of a light-emitting diode, a laser diode, and a light-emitting element for outputting a light beam. The light beam 111 has a wavelength between 800nm to 900nm. The optical pattern 1111 is one form selected from the group consisting of a grid, a line, a cross, regular arranged points, and irregular arranged points. The object 3 is a dynamic object or a stationary object, or a combination of both. In one preferred embodiment of the present invention, a light-emitting diode (LED) is provided as the light source unit 11 at the front end of a care bed which is selected as the lying equipment 2. The light source unit 11 emits the light beam 111 with an optical pattern 1111 in the form of regularly arranged points on the surface of the care bed and the surrounding area, wherein the wavelength of the light beam 111 is between 800nm to 900nm and is optimal at 840nm. A dynamic object, such as an elderly lying on the care bed, causes a variation in shape of the optical pattern 111 projecting onto the care bed, whereas the front end of the lying equipment is the position of the elderly's head. It should be noted that, the positions of the light source unit 11, the composition, shape or number of the optical pattern 1111, and the types of the lying equipment 2 as mentioned above are only illustrative for explaining the aspects of the present invention, and not intended to limit the scope of the present invention. It is apparent to those skilled in the art that different positions of the light source units 11, different compositions, shapes or numbers of the optical pattern 1111, and different types of the lying equipment 2 will not affect the implementation of the present invention, as long as the shape variation can be visually identified from the images and the lying equipment 2 can be lain by the object 3.

The image capturing unit 12 is provided at a side of the light source unit 11, the image capturing unit 12 includes an optical lens 121 and an optical sensor 122 connected to the optical lens 121, wherein the optical lens 121 continuously captures a plurality of images of the object 3 and the optical pattern 1111 to form the images onto the optical sensor 122. The optical lens 121 is one device selected from the group consisting of a fisheye lens, a wide-angle lens and a standard lens. The optical sensor 122 is a charge coupled device (CCD) or a complementary metal-oxide-semiconductor (CMOS). In a preferred embodiment of the present invention, the image capturing unit 12 and the light source unit 11 are provided at the front end of the care bed, and the image capturing unit 12 and the light source unit 11 are spaced from each other by an appropriate distance. A fisheye lens selected as the optical lens 121 captures a plurality of images of the objects 3 and the optical pattern 1111 on the surface of the care bed and the surrounding area, to form the images onto a complementary metal-oxide-semiconductor (CMOS) selected as the optical sensor 122. Wherein the objects 3, for example, can be one selecting from the group consisting of elderly lying on the medical bed, a care provider in the surrounding area of the care bed, an outsider who breaks into the room, and a combination thereof, but does not limit thereto.

The image processing unit 13 is electrically connected to the image capturing unit 12, the image processing unit 13 includes a reception module 131 and a determination module 132 electrically connected to the reception module 131. The reception module 131 receives the plurality of images captured by the image capturing unit 12 and defines an image captured at an initial time to be an initial image P₀. The determination module 132 compares an image captured at a subsequent time with the initial image P₀, when the result of comparing indicates the images being inconsistent, the determination module 132 sends a signal. In a preferred embodiment of the present invention, the reception module 131 built in the image processing unit 13 receives the plurality of images captured by the image capturing unit 12 and sends the images to the determination module 132, wherein the reception module 131 defines the image captured at an initial time T₀ to be the initial image P₀, and the determination module 132 compares the image P₁ captured at a subsequent time T₁ with the initial image P₀, when the result of comparing indicates the images being inconsistent, the determination module 132 sends a signal. In other words, the determination module 132 compares the image P₁, which is captured at a time T₁ subsequent to the initial time T₀, with the initial image P₀. When there is a difference between the compared image P₁ and the initial image P₀, the determination module 132 sends a signal.

The warning unit 14 receives the signal sent by the determination unit 132 and sends a warning signal. The warning unit 14 is one device selected from the group consisting of an alarm, a display and a mobile communication device. The warning signal is presented by the warning unit as one type selected from the group consisting of a sound, a light, an image, a vibration and a combination thereof. The healthcare monitoring system 1 may further comprise a cloud server (not shown). The determination module 132, the cloud server and the warning unit 14 are interconnected with each other by a wireless transmission. The wireless transmission is one protocol selected from the group consisting of a bluetooth communication, an infrared communication, a near field communication (NFC), a wireless local area network (WLAN), a wireless gigabit alliance (WiGig), a ZigBee, a wireless universal serial bus (wireless USB), an ultra-wideband (UWB), a wireless fidelity (Wi-Fi), and a combination thereof. In a preferred embodiment of the present invention, the determination module 132 is electrically connected to the cloud server via a wireless local area network (WLAN) selected as the wireless transmission, and the cloud server is electrically connected to a mobile communication device selected as the warning unit 14 via the wireless local area network (WLAN). The warning signal generated after the determination module 132 compares and acquires a difference between the image P₁ and the initial image P₀ is transmitted to the mobile communication device via connection of the cloud server. The mobile communication device then notifies the owner of the mobile communication device about the elderly's condition by ways of sounds or images. The determination module 132 also transmits images with the difference to the mobile communication device.

In addition, the healthcare monitoring system 1 may further comprise a storage unit 15 electrically connected to the image capturing unit 12 to store the plurality of images captured by the image capturing unit 12. In a preferred embodiment of the present invention, the storage unit 15 is electrically connected to the image capturing unit 12 and the images captured by the image capturing unit 12 are stored in the storage unit 15 for utilization by the healthcare monitoring system 1 of the present invention or by the users.

Furthermore, the light source unit 11 may be a flashing light source of which a flashing frequency is the same as an image-capturing frequency of the image capturing unit 12, wherein the image processing unit 12 may further compare a former image and a latter image captured by the image capturing unit 12, and acquires the difference between the two images to determine variation of the optical pattern 1111. In a preferred embodiment of the present invention, the light source unit 11 is a flashing light source which illuminates and extinguishes alternatively, and the flashing frequency of the flashing light is the same as the image-capturing frequency of the image capturing unit 12. In other words, when the light beam 111 with the optical pattern 1111 emitted by the light source unit 11 illuminates, the image capturing unit 12 captures the image of the object 3 and the optical pattern 1111 and transmits the same to the image processing unit 13. At a later time after the light source 11 extinguishes, the image capturing unit 12 captures another image of the object 3 and transmits the same to the image processing unit 13, the image processing unit 13 compares the former image and the latter image captured by subtracting them from each other. Since the difference between the two images being the existence of the optical pattern 1111, the subtraction of the former and the latter images leaves out the image of the optical pattern 1111. The image processing unit 13 can therefore easily determines the variation in shape of the optical pattern 1111, to determine the condition of the object 3 that causes the variation in shape of the optical pattern 1111.

Next, in order to further comprehend the objective, features and the beneficial effects of the present invention, a specific embodiment according to the healthcare monitoring system 1 of the present invention is illustrated to show the range of actual application of the healthcare monitoring system 1 of the present invention, but not to limit the scope of the present invention in any aspects. When a care recipient is home alone and lying on a care bed as the lying equipment 2, the care recipient's family members or care providers can use the health monitoring system 1 of the present invention to effectively determine motions of the care recipient lying on the lying equipment 2, such as motions of getting up, turning over and hand waving, in addition to lying flat and detect activities of care providers or irrelevant persons around the lying equipment 2, by capturing the shape variation of the optical pattern 1111 emitted by the light source unit 11 provided at the surrounding of lying equipment 2 via the image capturing unit 12. The present invention overcomes the disadvantages that conventional care monitoring executed by an infrared transceiver or a gravity sensor is only capable of detecting certain set motions and that by a wearable sensor is merely allowed to analyze the vital signs of the care recipients, thereby having an advantage that the activities of the care recipient lying on the lying equipment 2 as well as the condition around the lying equipment 2 can be monitored comprehensively. First, a light source 11 unit is prepared at a side of a lying equipment 2, the light source unit 11 emits a light beam 111 including at least an optical pattern 1111 toward the lying equipment 2, wherein the optical pattern 111 is varied as it is projecting onto at least an object 3. Next, an image capturing unit 12 is prepared at a side of the light source unit 11, the image capturing unit 12 includes an optical lens 121 and an optical sensor 122 connected to the optical lens 121, wherein the optical lens 121 continuously captures a plurality of images of the object 3 and the optical pattern 1111 to form images onto the optical sensor 122. Next, an image processing unit 13 is prepared to electrically connect to the image capturing unit 12, the image processing unit 13 includes a reception module 131 and a determination module 132 electrically connected to the reception module 131. The reception module 131 receives the plurality of images captured by the image capturing unit 12 and defines the image captured at an initial time to be an initial image P₀. The determination module 132 compares the image captured at a subsequent time with the initial image P₀, when the result of comparing indicates the images being inconsistent, the determination module 132 sends a signal. Finally, a warning unit 14 is prepared to receive the signal sent by the determination unit 132 and send a warning signal. As the hardware of the healthcare monitoring system 1 of the present invention is completely set up, the optical lens 121 captures a plurality of images of the care recipients lying on the care bed, the objects 3 in the surrounding area and the optical pattern 1111, to form the images onto the optical sensor 122 and transmits the images to the reception module 131 of the image processing unit 13. The reception module 131 defines the image captured at an initial time T₀ to be an initial image P₀; that is, the initial image P₀ is the image of the care recipient lying on the lying equipment 2. Then, the determination module 132 compares the image P₁ captured at a subsequent time T₁ with the initial image P₀, the image P₁, for example, can be images of the motions of hand waving, getting up, or leaving the bed of the care recipients, but is not limited thereto. These motions results in variations of the optical pattern 1111 projecting on the body of the care recipient. When the determination module 132 compares the initial image P₀ with the image P₁, and the result of comparing indicates the images being inconsistent, the determination module 132 sends a signal to a mobile communication device selected as the warning unit 14. The owner of the mobile communication device is then notified of the condition of the elderly by ways of sounds or images via the mobile communication device. The determination module 132 also transmits the image with the difference to the mobile communication device. Besides, since the optical pattern 1111 of the present invention covers the bed surface of the lying equipment 2 and the surrounding area, the occurrence of an irrelevant person approaching adjacent to the lying equipment 2 will result in variation in shape of the optical pattern 111. The determination module 132 determines the variation and then sends a signal notifying relevant persons a condition of invasion by irrelevant persons.

According to the illustration above, the healthcare monitoring system of the present invention has the following beneficial effects comparing to the conventional art:
1. The healthcare monitoring system of the present invention effectively determines motions of the care recipient lying on the lying equipment, such as motions of getting up, turning over and hand waving, in addition to lying flat, and detects activities of care providers or irrelevant persons around the lying equipment, mainly by capturing the shape variation of the optical pattern emitted by the light source unit provided at the surrounding of the lying equipment via the image capturing unit, thereby having advantages that the activities of the care recipient lying on the lying equipment as well as the condition around the lying equipment can be monitored comprehensively and that the set up cost of the system is reduced.
2. The healthcare monitoring system of the present invention overcomes the disadvantages that conventional care monitoring executed by an infrared transceiver or a gravity sensor is only capable of detecting certain set motions, and that by a wearable sensor is merely allowed to analyze the vital signs of the care recipients, thereby having advantages that the motions and conditions of all dynamic objects within a spacious room can be monitored and that the monitoring is free from errors.
3. The healthcare monitoring system of the present invention provides effective light source for the image capturing unit at a midnight environment where no light is given for illumination, by setting up hardware in which the light source is capable of emitting the optical pattern in infrared light, such that the variation in shape of the optical pattern can be captured by visual means of images, thereby having advantages in achieving all day monitoring of the care recipient's actions and conditions, and executing all day security monitoring in the room.

Although the present invention has been described with reference to the preferred embodiments thereof, it is apparent to those skilled in the art that a variety of modifications and changes may be made without departing from the scope of the present invention which is intended to be defined by the appended claims.

## Claims

1. A healthcare monitoring system, at least comprising:
a light source unit provided at a side of a lying equipment, the light source unit emitting a light beam including at least an optical pattern toward the lying equipment, wherein the optical pattern is varied as projecting onto at least an object,
an image capturing unit provided at a side of the light source unit, the image capturing unit including an optical lens and an optical sensor connected to the optical lens, wherein the optical lens continuously captures a plurality of images of the object and the optical pattern to form the images onto the optical sensor, the light source unit is a flashing light source of which a flashing frequency is the same as an image-capturing frequency of the image capturing unit,
an image processing unit electrically connected to the image capturing unit, the image processing unit including a reception module and a determination module electrically connected to the reception module, the reception module receiving the plurality of images captured by the image capturing unit and defining the image captured at an initial time to be an initial image, the determination module comparing the image captured at a subsequent time with the initial image, when the result of comparing indicating the images being inconsistent, the determination module sending a signal, and
a warning unit receiving the signal sent by the determination unit and sending a warning signal.

2. The healthcare monitoring system according to claim 1, further comprising a storage unit electrically connected to the image capturing unit to store the plurality of images captured by the image capturing unit.

3. The healthcare monitoring system according to claim 1, wherein the lying equipment is one selected from the group consisting of a home bed, a medical bed, an electric bed, a care bed, a flat bed, a Japanese bed, an air-cushioned bed, a futon, a baby cot, a sofa and a long bench.

4. The healthcare monitoring system according to claim 1, wherein the light source unit is one selected from the group consisting of a light-emitting diode, a laser diode, and a light-emitting element for outputting a light beam.

5. The healthcare monitoring system according to claim 1, wherein the light beam has a wavelength above 800nm.

6. The healthcare monitoring system according to claim 2, wherein the light beam has a wavelength between 1000nm to 1500nm.

7. The healthcare monitoring system according to claim 1, wherein the optical pattern is one selected from the group consisting of a grid, a line, a cross, regular arranged points, and irregular arranged points.

8. The healthcare monitoring system according to claim 1, wherein the object is a dynamic object or a stationary object, or a combination of both.

9. The healthcare monitoring system according to claim 1, wherein the optical lens is one selected from the group consisting of a fisheye lens, a wide-angle lens and a standard lens.

10. The healthcare monitoring system according to claim 1, wherein the optical sensor is a charge coupled device (CCD) or a complementary metal-oxide-semiconductor (CMOS).

11. The healthcare monitoring system according to claim 1, wherein the warning unit is one selected from the group consisting of an alarm, a display and a mobile communication device.

12. The healthcare monitoring system according to claim 1, wherein the warning signal is presented by the warning unit as one selected from the group consisting of a sound, a light, an image, a vibration, and a combination thereof.

13. The healthcare monitoring system according to claim 1, further comprising a cloud server, wherein the determination module, the cloud server and the warning unit are interconnected with each other by a wireless transmission.

14. The healthcare monitoring system according to claim 12, wherein the wireless transmission is one selected from the group consisting of a bluetooth communication, an infrared communication, a near field communication (NFC), a wireless local area network (WLAN), a wireless gigabit alliance (WiGig), a ZigBee, a wireless universal serial bus (wireless USB), an ultra-wideband (UWB), a wireless fidelity (Wi-Fi), and a combination thereof.

15. The healthcare monitoring system according to claim 14, wherein the image processing unit further compares a former image and a latter image captured by the image capturing unit, and acquires the difference between the two images to determine variation of the optical pattern.
